(19) Europäisches Patentamt
European Patent Office
Office européen des brevets

(11) EP 1 259 263 B1

(12) **EUROPÄISCHE PATENTSCHRIFT**

(45) Veröffentlichungstag und Bekanntmachung des Hinweises auf die Patenterteilung:
**15.09.2004 Patentblatt 2004/38**

(21) Anmeldenummer: **00990682.7**

(22) Anmeldetag: **08.12.2000**

(51) Int Cl.$^7$: **A61K 47/48**, C12N 15/87, C12N 9/00

(86) Internationale Anmeldenummer:
**PCT/EP2000/012413**

(87) Internationale Veröffentlichungsnummer:
**WO 2001/043777 (21.06.2001 Gazette 2001/25)**

(54) **KOMPLEXIERUNG VON RNS, INSBESONDERE VON RIBOZYMEN, MIT POLYETHYLENIMINEN ZU DEREN STABILISIERUNG UND ZELLULÄREN EINSCHLEUSUNG**

COMPLEXATION OF RNA, ESPECIALLY RIBOZYMES, WITH POLYETHYLENIMINES FOR THE STABILIZATION AND CELLULAR INTRODUCTION THEREOF

COMPLEXATION D'ARN, NOTAMMENT DE RIBOZYMES, AVEC DES POLYETHYLENIMINES POUR SA STABILISATION ET SON INTRODUCTION CELLULAIRE

(84) Benannte Vertragsstaaten:
**AT BE CH CY DE DK ES FI FR GB GR IE IT LI LU MC NL PT SE TR**

(30) Priorität: **14.12.1999 DE 19960206**

(43) Veröffentlichungstag der Anmeldung:
**27.11.2002 Patentblatt 2002/48**

(73) Patentinhaber: **Indivumed GmbH**
**22297 Hamburg (DE)**

(72) Erfinder:
- **Aigner, Achim**
  **63069 Offenbach (DE)**
- **Czubayko, Frank**
  **35043 Marburg (DE)**
- **Kissel, Thomas, Prof. Dr.**
  **79219 Staufen (DE)**
- **Fischer, Dagmar**
  **35037 Marburg (DE)**

(74) Vertreter: **Walcher, Armin et al**
**Louis, Pöhlau, Lohrentz & Segeth**
**Postfach 3055**
**90014 Nürnberg (DE)**

(56) Entgegenhaltungen:

**EP-A- 0 248 531**
**WO-A-00/59548**
**WO-A-96/02655**
**WO-A-98/59064**
**WO-A-99/05303**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 259 263 B1

Printed by Jouve, 75001 PARIS (FR)

## Beschreibung

Die Erfindung betrifft die extra- und intrazelluläre Stabilisierung von modifizierten oder nicht-modifizierten RNS-Molekülen, insbesondere von Ribozymen, gegen enzymatischen und nicht-enzymatischen Abbau durch Komplexierung mit auf Polyethylenimin (PEI) basierenden Makromolekülen sowie die Einschleusung dieser Komplexe in Zellen unter nachfolgender Freisetzung biologisch aktiver RNS-Moleküle.

Aus WO-A-96/02655 sind Komplexe von Desoxyribonukleinsäuren (DNS) mit Polyethyleniminen mit einem durchschnittlichen Molekulargewicht von 50 kDa bzw. 800 kDa und deren Verwendung in der Gentherapie beschrieben.

In WO-A-98/59064 werden Komplexe von DNS mit modifizierten Polyethyleniminen mit einem mittleren Molekulargewicht von 800 kDa und deren Verwendung für den Gentransfer in Säugerzellen beschrieben.

In M.A.Reynolds (Exogenous Delivery of Ribozymes, in: Scanlon, K.J. und Kashani-Sabet, M. (eds.) *Ribozymes in the Gene Therapy of Cancer,* pp. 41-60. R.G. Landes Company, 1998) wird auf die nicht publizierte Beobachtung hingewiesen, dass unter Verwendung nicht näher beschriebener Fluoreszenzfarbstoff-markierter Ribozyme in der Zellkultur mit einem nicht näher beschriebenen PEI eine mehr als 70%ige nukleäre Fluoreszenzmarkierung von Zellen erzielt werden konnte.

Ribonukleinsäure üben in der Zelle vielfältige Funktionen aus und werden entsprechend u.a. als messenger RNS (mRNS), transfer RNS (tRNS), ribosomale RNS (rRNS) bezeichnet.

Von besonderer biomedizinischer Bedeutung sind bestimmte spezialisierte RNS-Moleküle, die sogenannten Ribozyme. Ribozyme sind katalytisch aktive, kleine RNS-Moleküle, die in der Lage sind, andere, für essentielle Zellfunktionen bedeutsame RNS-Moleküle sequenzspezifisch zu spalten. Es kommt damit zur Herunterregulation der Expression des jeweiligen Gens, d.h. zur Hemmung spezifischer Genfunktionen. Entsprechend ihrer Struktur, Funktion, Aktivität und Grösse lassen sich verschiedene Ribozyme bzw. Ribozymklassen unterscheiden (vgl. Tab. 1). Evtl. assoziierte Proteinkomponenten dienen dabei der Stabilisierung und Effizienzerhöhung und sind nicht der eigentliche Träger der katalytischen Aktivität.

| Ribozyme | Aktivität | Funktion | Größe |
|---|---|---|---|
| Hammerhead-Motiv | Ribonuklease (5´OH) | Replikation | ≈ 35 Nukleotide |
| Hairpin-Motiv | Ribonuklease (5´OH) | Replikation | ≈ 60 Nukleotide |
| HDV Ribozym | Ribonuklease (5´OH) | Replikation | ≈ 80 Nukleotide |
| RNase P | Ribonuklease (5´P) | pre-tRNA Spaltung | > 275 Nukleotide |
| Intron Gruppe 1 | Umesterung | Spleißen | > 200 Nukleotide |
| Intron Gruppe 2 | Umesterung | Spleißen | > 600 Nukleotide |

*Tab. 1: Zusammenfassung der verschiedenen Ribozyme mit ihrer Aktivität, Funktion und Größe.*

*Ribonuklease (5´OH) steht für eine Ribonukleaseaktivität, die ein 5´-OH und einen 2´-3´-zyklischen Phosphatrest erzeugt, Ribonuklease (5´P) steht für eine Reaktion, bei der ein 5´-Phosphat und ein 3´-OH-Ende entsteht.*

Die Hammerhead-Ribozyme gehören zu den kleinsten und am besten erforschten katalytischen RNS-Molekülen und sind in der Lage, andere RNS-Moleküle sequenzspezifisch zu spalten. Das am häufigsten benutzte Hammerhead-Ribozym ist von dem Original-Ribozym nach Haseloff-Gerlach abgeleitet (Nature 334, 585-59 (1988)), das modifiziert und auf ein 22 Nukleotide umfassendes katalytisches Zentrum reduziert wurde. Diese Kernsequenz, dessen dreidimensionale und katalytisch aktive Struktur durch Watson-Crick-Basenpaarung entsteht, wird von zwei Armen flankiert, die ebenfalls aufgrund einer Watson-Crick-Basenpaarung spezifisch an den komplementären Sequenzabschnitt der Ziel-RNS hybridisieren und somit den katalytisch aktiven Bereich des Ribozyms in hinreichende räumliche Nähe zu der Spaltstelle der Ziel-RNS bringen. Durch spezifische Spaltung mit anschließendem zellulären Abbau von RNS-Molekülen bestimmter Gene können Ribozyme so u.a. zur diagnostischen und therapeutischen Hemmung spezifischer Genfunktionen genutzt werden.

Die größten Hindernisse, die bislang für den Einsatz von Ribozymen überwunden werden müssen, sind die sehr hohe Anfälligkeit der Moleküle gegenüber dem enzymatischen Abbau durch ubiquitär präsente intra- und extrazelluläre RNS-abbauende Enzyme (Ribonukleasen, RNasen) sowie die schlechte Penetration der Ribozyme in Zellen.

Während intrazellulär produzierte, natürliche RNS-Moleküle (z.B. mRNS und tRNS) in der intakten Zelle durch strukturspezifische Eigenschaften und zelluläre Modifikationen unter bestimmten Bedingungen zumindest über eine begrenzte Stabilität verfügen, lassen sich synthetische RNS-Moleküle wie Ribozyme nur durch chemische Modifikationen gegen diesen schnellen Abbau schützen. Diese chemischen Modifikationen sind jedoch mit wesentlichen Nachteilen behaftet:

- Funktionseinschränkung bis hin zum vollständigen Verlust der Aktivität des Ribozyms

- Entstehung von modifizierten Ribozymen mit ungünstigen, insbesondere zelltoxischen Eigenschaften
- Sehr hoher Aufwand zur Entwicklung dieser chemischen Modifikationen und deren chemischer Umsetzung

Trotzdem sind aufgrund der potentiell überragenden therapeutischen Bedeutung von Ribozymen zahlreiche Anstrengungen zur Stabilisierung durch chemische Modifikationen unternommen worden (z.B. Heidenreich et al., J. Biol. Chem. 269, 2131-2138 (1994); Pieken et al., Science 253, 314-317 (1991)). Allerdings gilt auch hier, dass diese Modifikationen erstens meist zur Inaktivierung des Ribozyms führen, und zweitens eine sehr aufwendige und teure Synthese erfordern.

Zusammenfassend kann man sagen, dass ein effektives Verfahren, welches

- RNS-Moleküle vor Degradation schützt, jedoch
- die Struktur der Moleküle möglichst wenig verändert, sowie
- deren Funktion möglichst nicht beeinflusst,

von großem grundlagen- und anwendungsorientiertem wissenschaftlichem und wirtschaftlichem Interesse ist.

in der vorliegenden Erfindung wird beschrieben, dass RNS-Moleküle, insbesondere Ribozyme, durch Komplexierung mit auf Polyethylenimin (PEI) basierenden Makromolekülen extra- und intrazellulär vor enzymatischem und nicht-enzymatischem Abbau geschützt werden können. Sogar Ribozyme, die sonst einer besonders raschen Degradation unterliegen, werden dabei vollständig vor Abbau geschützt, ohne dass ein Aktivitätsverlust eintritt. Die PEI-Ribozym-Komplexe sind selbst in biologischen, serumhaltigen Medien stabil. Weiterhin entsteht ein Komplex mit geringer Toxizität, der in der Lage ist, Ribozyme sehr effizient in funktionell aktiver Form in lebende Zellen einzuschleusen und somit zelluläre Prozesse in der gewünschten Form zu manipulieren. Über Ribozyme hinausgehend gestattet die Komplexierung von RNS schließlich eine viel weitergehende, vielfältige Verwendung zur Stabilisierung verschiedenster RNS-Moleküle.

So ist die Methode zusammenfassend dargestellt geeignet

- zur Stabilisierung von RNS-Molekülen, insbesondere Ribozymen, vor enzymatischem oder nicht-enzymatischem Abbau *in vitro* und *in vivo,* extrazellulär oder intrazellulär.
- zur Stabilisierung bei der Lagerung von RNS-Molekülen, insbesondere Ribozymen.
- zur Stabilisierung beim Transport von RNS-Molekülen, insbesondere Ribozymen.
- zur Stabilisierung von RNS-Molekülen, insbesondere von Ribozymen, bei deren Einsatz in Laborexperimenten
- als Vektor für RNS-Moleküle, insbesondere für Ribozyme in Gentransferanwendungen in vivo (Gentherapie)
- als Vektor für RNS-Moleküle, insbesondere für Ribozyme, zur Anwendung in Zellkultur-Experimenten

Die Erfindung betrifft daher Komplexe aus RNS, insbesondere Ribozymen, und Polyethylenimin (PEI), in welchen das PEI ein Molekulargewicht von 700, vorzugsweise 1.400, bis 25.000 Da, insbesondere 1.600 bis 15.000 Da aufweist. Bevorzugt sind erfindungsgemäße Komplexe, in welchen das molare Verhältnis der Stickstoffatome im PEI zu den Phosphoratomen in der RNS (N/P-Wert) 1 bis 100, vorzugsweise 2 bis 40, insbesondere 3 bis 10 beträgt.

In den erfindungsgemäßen Komplexen kann PEI durch zelluläre Liganden modifiziert sein. Diese Modifikation kann vor oder nach der Komplexierung der RNS erfolgen. Ebenso kann eine Modifikation durch hydrophilen Polymere wie z.B. Polyethylenglykol (PEG) mit einem Molekulargewicht von 100 bis 10.000.000 g/mol, vorzugsweise von 1.000 bis 100.000 g/mol und insbesondere von 5.000 bis 50.000 g/mol erfolgen. Dabei kann ein PEI durch ein oder mehrere hydrophile Polymere oder ein hydrophiles Polymere durch ein oder mehrere PEIs modifiziert sein. Solche modifizierten PEIs und Verfahren zu ihrer Herstellung sind beschrieben in der internationalen Anmeldung PCT/EP00/06214.

Die zu schützende RNS kann jede beliebige RNS, insbesondere eine messenger-RNS (mRNS), eine ribosomale RNS (rRNS), eine transfer-RNS (tRNS), eine Kern-RNS (nukleäre RNS), ein RNS-Ribozym, eine in vitro transkribierte RNS oder eine chemisch synthetisierte RNS sein, wobei alle genannten RNS-Moleküle chemisch modifiziert sein können. Dabei kann die RNS 10 bis 10.000 Nukleotide, vorzugsweise 15 bis 1.500 Nukleotide, insbesondere 20 bis 1.000 Nukleotide aufweisen. Im Falle von Ribozymen sind Kettenlängen von 20 - 600 Nukleotiden, insbesondere von 35 - 80 Nukleotiden, bevorzugt.

Bevorzugt sind Komplexe, in welchen die RNS ein Ribozym, insbesondere ein Hammerhead- oder ein Hairpin-Ribozym, ist oder bei der es sich um RNS-Aptamere oder Antisense-Sequenzen handelt. Bevorzugt sind ferner Komplexe, in welchen die RNS ganz oder zum Teil für ein therapeutisch wirksames Peptid oder Protein oder aber für ein zelluläres Selbstmordgen kodiert.

Die Erfindung betrifft weiterhin ein Verfahren zur Herstellung eines erfindungsgemäßen Komplexes, bei welchem die RNS mit dem gegebenenfalls durch zelluläre Liganden oder durch weitere Polymere modifiziertem PEI durch Mischen der verdünnten Lösungen komplexiert wird.

Die Herstellung der erfindungsgemäßen Komplexe wird technisch wie folgt durchgeführt:

Für die Komplexierung von RNS, insbesondere von Ribozymen, mit PEI wird eine wässrige Lösung der RNS hergestellt. Die RNS kann chemisch modifiziert oder nicht modifiziert sein. Erfindungsgemäße Verfahren sind bevorzugt, bei welchen die RNS-Konzentration vorzugsweise 0,1 - 100 µg/ml, insbesondere 10 - 40 µg/ml beträgt.

Als Komplexierungsreagenzien sind käufliche Präparate modifizierten oder nicht modifizierten PEIs geeignet, sowie nicht käufliche modizifizierte oder nicht modifizierte PEIs wie z.B. LMW-PEI (Fischer et al. Pharm. Res. 16, 1273-9 (1999)). Zur Herstellung der jeweiligen Stammlösung wird das betreffende PEI in bidestilliertem Wasser gelöst und der pH-Wert mit etwa 100 mM HCl-Lösung auf einen neutralen pH-Wert vorzugsweise zwischen 7 und 8, besonders bevorzugt auf pH 7,4, eingestellt.

Unmittelbar vor der Durchführung der Komplexierungsreaktion wird die PEI-Stammlösung (bezüglich der eingesetzten Menge siehe untenstehende Tab. 2 u. 3) mit einer wässrigen etwa 150 mM neutralen NaCl-Lösung, pH 7 bis 8, vorzugsweise 7,4, auf das gewünschte Volumen eingestellt und einige Zeit, vorzugsweise mindestens 10 min bei RT inkubiert.

Die RNS (bezüglich der eingesetzten Menge siehe beispielsweise untenstehende Tab. 2 u. 3) wird mit einer wässrigen etwa 150 mM NaCl-Lösung, pH 7 bis 8, vorzugsweise 7,4, auf das gewünschte Volumen, besonders bevorzugt das etwa gleiche Volumen wie das der PEI-Lösung, gebracht und einige Zeit, vorzugsweise mindestens 10 min bei RT inkubiert.

Anschließend werden die PEI- und die RNS-Lösungen (Mischverhältnisse siehe beispielsweise untenstehende Tab. 2 u. 3) gemischt und einige Zeit, vorzugsweise mindestens 10 min bei RT inkubiert. Die Mischung kann dabei einmal oder mehrmals geschüttelt oder gevortext werden.

Die Komplexzusammensetzung und die Nettoladung werden auf der Basis von RNS-Phosphor zu PEI-Stickstoff berechnet und als Stickstoff-Phosphor-Verhältnis (N/P-Verhältnis, s.o.) nach Boussif et. al. (Proc. Natl. Acad. Sci. **92**, 7297-7301 (1995)) bzw. Phosphor-Stickstoff-Verhältnis angegeben.

Als Berechnungsgrundlage dient folgende Formel:

$$1\ \mu g\ \text{RNS} = 3\ \text{nmol Phosphor}$$

sowie

$$1\ \mu l\ \text{PEI-Lsg.} = 10\ \text{nmol Stickstoff}$$

Die Ansatzverhältnisse eines Standardansetzes mit 2 µg RNS und den entsprechenden Mengen PEI werden in den folgenden Tabellen am Beispiel von zwei PEI-Präparaten zusammengefasst (siehe aber auch weitergehende Ausführungen unten):

| Ansatzverhältnisse RNS/HMW-PEI (Äquivalente) | Volumen HMW-PEI-Stammlösung 0,9mg/ml [µl] | HMW-PEI absolut [µg] |
|---|---|---|
| 1 + 1 | 6 (1:10 verdünnt) | 0,27 |
| 1 + 6,67 | 4 | 2,7 |
| 1 + 10 | 6 | 1,8 |
| 1 + 13,33 | 8 | 3,6 |
| 1 +20 | 12 | 5,4 |

*Tab.2: Übersicht über verwendbare HMW-PEI-Ansatzverhältnisse.*

| Ansatzverhälnisse RNS/LMW-PEI (Äquivalente) | Volumen LMW-PEI-Stammlösung 0,9mg/ml [µl] | LMW-PEI absolut [µg] |
|---|---|---|
| 1 + 1 | 6 (1:10 verdünnt) | 0,54 |
| 1 + 2 | 12 (1:10 verdünnt) | 1,08 |
| 1 + 13,33 | 8 | 7,2 |
| 1 + 20 | 12 | 10,8 |
| 1 + 26,67 | 16 | 14,4 |
| 1 + 40 | 24 | 21,6 |
| 1 + 66,66 | 40 | 36 |
| 1+ 100 | 60 | 54 |

*Tab. 3: Übersicht über verwendbare LMW-PEI-Ansatzverhältnisse.*

Nach erfolgter Inkubation sind die Komplexe gebrauchsfertig verwendbar bzw. können in weitere chemische Modifikationen eingesetzt werden.

Die Erfindung betrifft die dargestellten Verfahren zum Schutz von RNS, insbesondere von Ribozymen, gegen enzymatischen und nicht-enzymatischen Abbau, bei welchen die RNS-Moleküle im Komplex mit modifiziertem oder nicht-modifiziertem PEI stabilisiert werden, sowie Verfahren bei welchen RNS-Ribozyme vor dem Abbau durch Nukleasen geschützt werden.

Ein weiterer Aspekt der Erfindung sind Verfahren zur Einschleusung von RNS, insbesondere Ribozyme, in Zellen, vorzugsweise in eukaryontische Zellen, und deren intrazelluläre Freisetzung in biologisch aktiver Form.

Die Erfindung betrifft ferner Zusammensetzungen, welche eine wirksame Menge mindestens eines erfindungsgemäßen RNS-PEI-Komplexes enthält, wobei die RNS in jeder für Zellen oder für den jeweiligen Organismus subtoxischen oder zumindest bei mehrstündiger Behandlung noch nicht dauerhaft zellschädigender Konzentration vorliegen kann.

Die Erfindung betrifft außerdem pharmazeutische Zusammensetzungen enthaltend eine wirksame Menge mindestens eines erfindungsgemäßen RNS-PEI-Komplexes und einen oder mehrere physiologisch unbedenkliche Träger.

Das RNS/PEI-Verhältnis lässt sich über das molare Verhältnis der Stickstoffatome im PEI zu den Phosphoratomen in der RNS angeben (N/P-Wert). Der N/P-Wert darf für eine vollständige Komplexierung der RNS nicht zu niedrig sein.

Vor allem nach oben kann der N/P-Wert der Komplexe aber über einen breiten Bereich schwanken, er kann im Bereich von etwa 1 bis etwa 100 gelegen sein. Bevorzugt beträgt das Verhältnis etwa 2 bis etwa 40, besonders bevorzugt beträgt das Verhältnis 3 bis 10.

Für den speziellen Anwendungsfall, z.B. für den jeweiligen RNS-Typ, insbesondere für Ribozyme, für PEI bestimmter Molmassen oder für den jeweiligen Zelltyp, können optimale N/P-Werte durch Vorversuche ermittelt werden. Bei schrittweiser Erhöhung des Verhältnisses unter ansonsten identischen Bedingungen sind dabei der Umfang des Schutzes der RNS, insbesondere des Ribozyms, die Effizienz des RNS-, insbesondere des Ribozym-Transfers, die intrazelluläre Aktivität des Ribozyms und das Ausschließen toxischer Wirkungen des Komplexes oder einzelner Bestandteile die wichtigsten Parameter, evtl. auch Materialersparnis oder Zeitkinetiken .

Das hier für die Komplexierung verwendete PEI weist Molekulargewichte zwischen 700 und 1.000.000 Da auf. Über den gesamten Molekulargewichtsbereich sowie unabhängig vom Verzweigungsgrad sind alle PEI-Moleküle, modifiziert oder nicht modifiziert, zum Schutz von RNS-Molekülen, insbesondere von Ribozymen, geeignet, weisen aber Unterschiede bezüglich der zellulären Aufnahme der Komplexe auf. Große PEI-Moleküle zeigen bereits bei niedrigen N/P-Verhältnissen eine optimale Effizienz bei der zellulären Aufnahme, sind aber toxischer als kleine PEI-Moleküle. Letztere erfordern bei gleicher RNS-Menge, insbesondere Ribozym-Menge, zur Komplexierung ein höheres N/P-Verhältnis, ohne dabei vergleichbar toxisch zu sein. Welches PEI-Molekül im einzelnen verwendet wird, kann in Vorversuchen ermittelt werden.

Sie lassen sich beispielsweise durch die Strukturformel I beschreiben,

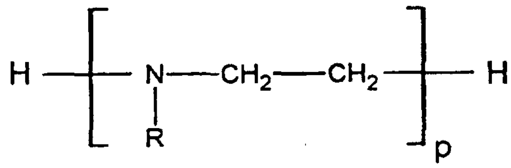
(I)

worin R ein Rest der Formel II ist,

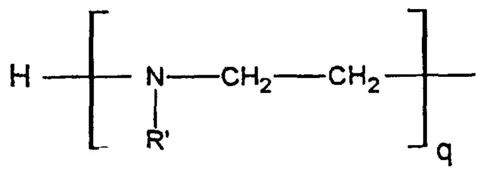
(II)

in welchem R' Wasserstoff bedeutet. p ist eine positive ganze Zahl, q ist 0 oder eine positive ganze Zahl und (p+ q) ist 15 - 25.000, vorzugsweise 30 - 600, insbesondere 35 - 400. Darüber hinaus kann II auch verzweigt sein. Dann ist R' ein Rest eines PEI, der analog wie Formel II aufgebaut ist.

Bevorzugt im Rahmen der vorliegenden Erfindung sind PEI-Moleküle im Molekulargewichtsbereich zwischen 1.400 und 25.000.

Hierzu ist LMW-PEI ("low molecular weight PEI") geeignet, das aus Aziridin (Ethylenimin-Monomer) durch ringöffnende Polymerisation in wässriger Lösung unter Säurekatalyse hergestellt wird. Die Synthese wird in Fischer et al., Pharm. Res. **16** , 1273-9 (1999) beschrieben.

Daneben können kommerziell erhältliche PEI-Präparationen mit unterschiedlichen Molekulargewichten eingesetzt werden. Beispiele sind PEI 700 Da, PEI 2000 Da und PEI 25000 Da (Aldrich), sowie, von der Firma BASF unter dem Markennamen Lupasol® angeboten, 800 Da (Lupasol® FG), 1300 Da (Lupasol® G 20 wasserfrei), 1300 Da (Lupasol® G 20), 2000 Da (Lupasol® G 35) und 25000 Da (Lupasol® WF).

Das PEI kann durch einen zellulären Liganden, im folgenden mit "Q" bezeichnet, modifiziert sein. Unter einem zellulären Liganden versteht man eine Gruppe, die eine spezifische oder unspezifische biologische Funktion ausübt, insbesondere einen Bindungspartner darstellt für Wechselwirkungen mit Rezeptoren oder anderen zellulären Proteinen. Die Modifikation mit einem Liganden dient insbesondere der Zielzell-spezifischen Aufnahme eines RNS-PEI-Q-Komplexes in höhere eukaryontische Zellen und deren Zellkern, wobei die RNS vorzugsweise ein Ribozym (Gentargeting) ist.

Q kann somit auch ein Ligand für eine spezifische Wechselwirkung und für die bevorzugte Aufnahme der RNS-PEI-Q-Komplexe in Zielzellen, -gewebe oder - organe sein. Beispiele für Q sind Proteine, insbesondere

- Antikörper oder Antikörperfragmente wie Fab, $F(ab_2)$, scFv, oder
- Cyto- oder Lymphokine, wie Interleukine (IL-2 bis X), Interferon GM-CSF, oder
- Wachstumsfaktoren, wie EGF, PDGF, FGF, EPO, oder
- Integrine wie ICAM, VCAM, oder
- Glykoproteine wie Lektine oder glykosylierte Proteine (s.o.), oder
- Lipoproteine wie LDL, HDL, oder
- Transporterproteine wie Transferrin, oder
- Peptide wie LH-RH, Calcitonin, Oxytocin, Insulin, Somatostatin, IGF, RGD, oder
- Kohlenhydrate wie Galactose, Mannose, Glucose, Lactose, oder
- Hormone wie Steroide, THR, oder
- Vitamine wie Vitamin B12, Folsäure.

Für bestimmte *in vivo* Anwendungen ist es im Hinblick auf eine hohe Gentransfereffizienz erforderlich, dass die erfindungsgemäßen Komplexe in hoher Konzentration vorliegen. Die erfindungsgemäßen Komplexe weisen den Vorteil auf, dass sie aus verdünnten Lösungen beispielsweise über physikalische Methoden wie Ultrazentrifugation, Ultrafiltration oder Druckdialyse auf die erforderliche hohe Konzentration gebracht werden können, ohne dass eine nennenswerte Aggregatbildung oder sonstige Veränderung der Komplexe, die die Gentransfereffizienz beeinträchtigen würde, auftritt.

Insbesondere liegt die Zusammensetzung in Form einer pharmazeutischen Zusammensetzung vor. In dieser Ausgestaltung dient die Zusammensetzung nicht nur zum Schutz von RNS, sondern auch zum Transfer der RNS in Zellen, insbesondere eukaryontischen Zellen, besonders bevorzugt Säugetierzellen, *in vivo* oder *in vitro.* Sie enthält als aktiven Bestandteil einen Komplex, der eine therapeutisch wirksame RNS, insbesondere ein Ribozym oder mehrere Ribozyme, enthält. Mit Hilfe der erfindungsgemäßen pharmazeutischen Zusammensetzung kann, bei lokaler Anwendung, eine hohe Konzentration an therapeutisch wirksamer RNS in den Zielzellen bzw. bei *in vivo*-Experimenten im Zielgewebe erzielt werden. Bei der systemischen Anwendung hat die Zusammensetzung den Vorteil, dass die Komplexe wegen der Verhinderung der Opsonierung weder unspezifischer Bindung noch Immunsystem-induziertem Abbau unterliegen.

Durch Verhinderung bzw. Verringerung von unspezifischen Bindungen bei gleichzeitigem Einführen von (zelltypspezifischen) zellbindenden Liganden in die Komplexe kann ein spezifisches Targeting zu bestimmten Zellen, Organen oder Geweben und damit eine zielgerichtete Genexpression (z.B. in Tumorgewebe) oder andere Wirkung nach systemischer Verabreichung erzielt werden.

In Geweben mit erhöhter Gefäßpermeabilität bzw. mit Gefäßschädigungen können die erfindungsgemäßen RNS-PEI-Komplexe aus der Blutbahn in die umliegenden Gewebe austreten und dort akkumulieren. Bereiche, wo ein solches "passives Targeting" verstärkt auftritt, sind z.B. gut durchblutete Tumore sowie Entzündungsbereiche. In manchen dieser Fälle wird dann bereits eine hinreichend hohe Zielselektivität erreicht werden, so dass die Verknüpfung von PEI mit zielzellspezifischen Liganden unterbleiben kann.

Die pharmazeutische Zusammensetzung kann u.a. vorteilhaft für die Therapie von Tumorerkrankungen verwendet werden, um intratumoral oder systemisch RNS, insbesondere Ribozyme, zu verabreichen.

Eine weitere Anwendung, bei der die Vorteile der erfindungsgemäßen. Zusammensetzung zum Tragen kommen, ist die sog. genetische Tumorvakzinierung. Die dabei benutzten Komplexe enthalten RNS, kodierend für ein oder mehrere Tumorantigene oder Fragmente davon.

Die erfindungsgemäße pharmazeutische Zusammensetzung des Komplexes liegt bevorzugt als Lyophilisat vor, gegebenenfalls unter Zusatz von Zucker wie Saccharose oder Dextrose in einer Menge, die in der gebrauchsfertigen Lösung eine physiologische Konzentration ergibt. Die erfindungsgemäße Zusammensetzung kann auch tiefgefroren (kryokonserviert) oder als gekühlte Lösung vorliegen und weitere Zusätze enthalten.

Die erfindungsgemäßen Zusammensetzungen können gegebenenfalls in Form eines Kits vorliegen, wobei die Einzelkomponenten RNS, insbesondere Ribozyme, einerseits und PEI, an das gegebenenfalls ein Ligand gekoppelt ist, andererseits in getrennten Behältern vorliegen.

Die folgenden Beispiele dienen zur Erläuterung der Erfindung, ohne dass diese darauf beschränkt wäre.

**Beispiel 1:**

**Schutz von nicht-modifizierten, chemisch synthetisierten RNS-Oligonukleotiden (37 Nukleotide) vor enzymatischem Abbau durch Komplexierung mit PEI.**

Die in der Literatur bisher nicht beschriebene Komplexierung zwischen Hammerhead-Ribozymen und PEI wurde in Bezug auf die Stabilität des Ribozyms gegenüber der Degradation durch Serumnukleasen untersucht. Eine so erzielte Verlängerung der Halbwertzeit in Anwesenheit von Serum würde eine systemische Applikation unmodifizierter Ribozyme ermöglichen, die ihren chemisch modifizierten Analoga in Bezug auf katalytische Aktivität und Herstellungskosten überlegen sind.

Für die Versuche wurden zwei Polyethylenimin-Lösungen mit unterschiedlichem Molekulargewicht verwendet. HMW-PEI wurde als 50%ige wässrige Lösung von der Firma Fluka (Neu-Ulm) mit einem Molekulargewicht von 600-1000 kDa (Herstellerangabe, viskosimetrisch bestimmt) bezogen. Zur Herstellung der Stammlösung wurden 9,0 mg HMW-PEI in 8 ml bidestilliertem Wasser gelöst, der pH-Wert mit 0,1 N HCl-Lösung auf 7,4 eingestellt und mit bidestilliertem Wasser auf 10,0 ml aufgefüllt.

LMW-PEI wurde aus Aziridin (Ethylenimin-Monomer) durch ring-öffnende Polymerisation in wässriger Lösung unter Säurekatalyse hergestellt. Das Molekulargewicht von $\leq$ 10 kDa wurde mittels Laserstreulichtmessung von der Fa. Wellensieck (Minden) bestimmt (Fischer et al., Pharm. Res. **16**, 1273-9 (1999)). Zur Herstellung der Stammlösung wurden 9,0 mg LMW-PEI in 8 ml bidestilliertem Wasser gelöst, der pH-Wert mit 0,1 N HCl-Lösung auf 7,4 eingestellt und mit bidestilliertem Wasser auf 10,0 ml aufgefüllt.

Bei dem RNS-Oligonukleotid handelt es sich um ein Hammerhead-Ribozym mit einer Länge von 37 Basen, das mit einer 5'-O-DMT-ON-2'-Ofpmp-Schutzgruppe versehen war (Fa. MWG- Biotech GmbH, Ebersberg). Für die Stabilitätsuntersuchungen wurde die Schutzgruppe mittels eines mitgelieferten Schutzgruppenentfernungs-Kits (Fa.

Cruachem, Glasgow) durch saure Hydrolyse abgespalten und die entschützte RNS mittels Ethanolfällung gewonnen.

Die Komplexierung der Oligonukleotide mit PEI wurde nach der oben dargestellten Anleitung durchgeführt.

Es wurden für HMW-PEI fünf, für LMW-PEI sieben verschiedene Oligonukleotid-PEI-Verhältnisse gewählt. Die Auswahl erfolgte in Anlehnung an Komplexierungsversuche zwischen HMW- und LMW-PEI und dem cMV-nlacZ-Plasmid (Fischer, Nichtvirale Vektoren zur Gentherapie: Kationische Polymere als Vektorsyteme für den Transfer von DNA. Cuvillier Verlag, Göttingen, 1. Auflage (1998)).

Die Ansatzverhältnisse eines Standardansatzes mit 2 µg Oligo und den damit umgesetzten Mengen PEI werden in den folgenden Tabellen zusammengefasst:

| Ansatzverhältnisse RNS/HMW-PEI (N/P-Ratio) | Volumen HMW-PEI-Stammlösung 0,9mg/ml [µl] | HMW-PEI absolut [µg] |
|---|---|---|
| 1 + 1 | 6 (1:10 verdünnt) | 0,27 |
| 1 + 6,67 | 4 | 2,7 |
| 1 + 10 | 6 | 1,8 |
| 1 + 13,33 | 8 | 3,6 |
| 1 +20 | 12 | 5,4 |

*Tab.4:* *Übersicht über die verwendeten HMW-PEI-Ansatzverhältnisse.*

| Ansatzverhälnisse RNS/LMW-PEI (N/P-Ratio) | Volumen LMW-PEI-Stammlösung 0,9mg/ml [µl] | LMW-PEI absolut [µg] |
|---|---|---|
| 1 + 1 | 6 (1:10 verdünnt) | 0,54 |
| 1 + 2 | 12 (1:10 verdünnt) | 1,08 |
| 1 + 13,33 | 8 | 7,2 |
| 1 + 20 | 12 | 10,8 |
| 1 + 26,67 | 16 | 14,4 |
| 1 + 40 | 24 | 21,6 |
| 1 + 66,66 | 40 | 36 |

*Tab. 5:* *Übersicht über die verwendeten LMW-PEI-Ansatzverhältnisse.*

**Inkubation von Ribozym-PEI-Komplexen mit fötalem Kälberserum (FKS)**

Zur ersten Orientierung, ob PEI Ribozyme gegen den Abbau durch Serumnukleasen schützt, wurden die

Komplexe HMW-PEI 1+ 13,3 und LMW-PEI 1+ 66,6 gewählt und über 5 und 15 min bei 37°C in 100 % FKS inkubiert. Die Auswahl dieser beiden Komplexe erfolgte aufgrund der beschriebenen hohen Transfektionsraten von Plasmid-Komplexen im Vergleich zu den anderen HMW- und LMW-PEI-Komplexen ) (Fischer et al., Pharm. Res. **16**, 1273-9 (1999 )). Nach erfolgter Inkubation wurden die Serumproteine mittels Hitzeinaktivierung denaturiert, indem die Ansätze für 30 min in einem Wasserbad bei 70°C inkubiert wurden. Durch Zusatz von 8 µl 10%iger SDS-Lösung wurden noch aktive Enzyme zerstört und gleichzeitig die Ribozyme aus den Komplexen mit PEI freigesetzt. Die Mischung wurden anschließend gelelektrophoretisch aufgetrennt, und intakte RNS-Banden wurden mittels eines fluoreszierenden Cyanin-Farbstoffs (SYBR-Gold®, Fa. MoBiTech, Göttingen), der an einzel- oder doppelsträngige DNS oder RNS bindet, detektiert.

Im Gegensatz zu den nicht-komplexierten Ribozymen, die bereits nach 5 min vollständig degradiert waren, wurden die mit HMW-PEI oder LMW-PEI komplexierten Ribozyme über 15 min Inkubationsdauer fast vollständig vor dem Abbau durch die im Serum enthaltenen Nukleasen geschützt.

Es wurden nun HMW-PEI und LMW-PEI-Komplexe in den zuvor untersuchten Stickstoff-Phosphat-Verhältnissen hergestellt und für 15 - 120 min in 10 % Serum analog zum vorhergehenden Versuch inkubiert. Zur Kontrolle der Versuchsergebnisse wurden neben den nicht-komplexierten Ribozymen auch Ribozyme in den Komplexen HMW-PEI (1+1) und LMW-PEI (1 +2) mit aufgetragen, in denen wie aus Vorversuchen bekannt keine vollständige Komplexierung eintritt und die folglich keinen Schutz der RNS vor Degradation bieten sollten. Sowohl die nicht-komplexierten Ribozyme als auch die RNS-Oligonukleotide, die in zu niedrigen Stickstoff-Phosphat-Verhältnissen mit PEI komplexiert wurden, waren erwartungsgemäß sowohl nach 60 min als auch nach 120 min durch die Serumnukleasen vollständig degradiert. Die anderen beiden Komplexe (HMW-PEI 1 + 13,3 und LMW-PEI 1+ 66,6), die aufgrund einer vollständigen Komplexierung des Ribozyms durch PEI eine Schutzwirkung gegen den enzymatischen Abbau über einen Zeitraum von 30 min bereits gezeigt hatten, stabilisierten das Ribozym auch über 60 min und 120 min.

Abschließend kann somit festgehalten werden, dass HMW-PEI und LMW-PEI in der Lage sind, komplexierte RNS-Ribozyme ab Stickstoff-Phosphat-Verhältnissen von 1+6,67 (HMW-PEI) und 1+13,3 (LMW-PEI) gegen Degradation durch Serumnukleasen über einen Zeitraum von mindestens 120 min zu schützen. Diese Verlängerung der Halbwertzeit von wenigen Minuten auf mehr als zwei Stunden macht es möglich, nicht-modifizierte RNS-Ribozyme sowohl für *in-vitro* als auch *invivo* Transfektionsexperimente zu verwenden.

**Beispiel 2:**

**Schutz von nicht-modifizierten, in-vitro transkribierten RNS-Molekülen (300 - 800 Nukleotide) vor enzymatischem Abbau durch Komplexierung mit PEI.**

Wie in Beispiel 1 gezeigt, konnten kürzere RNS-Moleküle, die in ihrer Länge Hammerhead-Ribozymen oder tRNS-Molekülen entsprechen, durch PEI-Komplexierung stabilisiert werden. In einem zweiten Schritt wurde untersucht, ob auch längere RNS-Moleküle (bis zu 1000 Nukleotide), die mRNS-Molekülen entsprechen, in gleicher Weise geschützt werden. Da so lange RNS-Moleküle chemisch nicht synthetisiert werden können, wurde hier die Methode der *in-vitro* Transkription benutzt (in-vitro Trankriptionskit der Firma Promega). Hierbei wird ein DNS-Template (Plasmid-DNS) mittels RNS-Polymerase in vitro in RNS umgeschrieben. Diese RNS ähnelt in ihrer Länge und Struktur zellulärer mRNS, obwohl wichtige stabilisierende Elemente, wie die *5'-Cap* Struktur und der *3'-poly-A-Tail* fehlen. Somit ist *in vitro* trankribierte RNS empfindlicher gegen den Abbau durch Ribonukleasen als natürliche zelluläre mRNS. Zur besseren Darstellung und Quantifizierung wurde die RNS durch den Einbau $^{32}$P-markierter Nukleotide radioaktiv markiert. Für die Versuche wurden zwei verschiedene RNS-Moleküle hergestellt, die 300 bzw. 800 Nukleotide lang waren.

Die radioaktiv markierten RNS-Moleküle wurden, komplexiert mit LMW-PEI vs. nichtkomplexiert, entweder mit rekombinanter RNase A (Endkonzentration 20 ng/ml) oder fötalem Kälberserum (1% - 40 % Endkonzentration) für verschiedene Zeitintervalle (0 - 120 Minuten) inkubiert. Nach der Inkubation wurde die RNS auf einem denaturierenden Formaldehyd-Agarose-Gel elektrophoretisch aufgetrennt, und die RNS wurde anschließend mittels der Northern-blotting Technik auf eine RNS-bindende Nylonmembran transferiert. Zur qualitativen Auswertung wurden auf der getrockneten Membran Röntgenfilme für 2 - 8 d, zum Teil unter Verwendung eines Verstärkungssystems (BioMax), exponiert. Zur Quantifizierung wurden die radioaktiven Signale auf den Nylonmembranen über einen Phosphoimager der Firma Molecular Dynamics erfasst und mittels einer Analysesoftware derselben Firma ausgewertet.

Die Ergebnisse sind in Fig. 1 zusammengefaßt. Während nach der Inkubation mit RNase A (Endkonzentration 20 ng/ml) die nicht-komplexierte RNS erwartungsgemäß bereits nach 15 Minuten fast komplett abgebaut ist, sind von der PEI-komplexierten RNS-auch nach 2 Stunden Inkubation noch mehr als 60 % der Moleküle intakt. Eine sehr ähnliche Kinetik ergibt sich auch nach der Inkubation mit fötalem Kälberserum (Fig. 2). Hier ist die nicht-komplexierte RNS nach ca. 30 Minuten nach der Inkubation mit 1 % fötalem Kälberserum komplett abgebaut, während die PEI-komplexierte RNS auch nach 120 Minuten noch vollständig intakt ist. Auch bei Erhöhung der Konzentration auf 40 % fötales Kälberserum ist die PEI-komplexierte RNS gegenüber der nicht-komplexierten RNS deutlich stabiler. Die Ergebnisse

waren identisch für die verschieden langen RNS-Moleküle. Die Daten der Graphiken wurden aus zwei unabhängigen Experimenten gemittelt.

Zusammenfassend lässt sich feststellen, dass in vitro transkribierte RNS-Moleküle (300-800 Nukleotide lang) unkomplexiert erwartungsgemäß innerhalb sehr kurzer Zeit von Ribonukleasen abgebaut wurden. Durch Komplexierung mit niedermolekularem PEI (LMW-PEI) konnte die Halbwertzeit der RNS in fötalem Kälberserum (bis zu 40 % Endkonzentration) auf über zwei Stunden verlängert werden.

**Beispiel 3:**

**Chemisch-synthetisierte, nicht-modifizierte RNS-Ribozyme werden durch Komplexierung mit LMW-PEI in Tumorzellen eingeschleust und spalten ihre Ziel-RNS mit hoher Effizienz.**

Nachdem die ersten beiden Beispiele die Stabilisierung von verschiedenen RNS-Molekülen durch LMW-PEI demonstrierten, wurde untersucht, ob mit LMW-PEI komplexierte RNS in die Zellen gelangt und intrazellulär in ihrer biochemischen Funktion unbeeinträchtigt ist.

Zu diesem Zweck wurden die unter Beispiel 1 beschriebenen chemisch synthetisierten Hammerhead-Ribozyme verwendet. Diese Ribozyme sind gegen die humane mRNS eines Fibroblasten-Wachstumsfaktor-bindenden Proteins (FGF-BP) gerichtet (Czubayko et al., Nature Med. **3** (10), 1137-1140 (1997)). Das FGF-BP Protein wird in vielen menschlichen Tumoren und davon abgeleiteten Tumorzellinien exprimiert. Die Funktionalität der Ribozyme konnte in früheren Experimenten dadurch nachgewiesen werden, dass nach stabiler Transfektion der eukaryontischen Ribozymexpressionsvektoren in Tumorzellen (ME-180 Cervixkarzinomzellinie, LS174T Kolonkarzinomzellinie) das Zielgen auf mRNS- und auf Proteinebene deutlich reduziert wurde (Czubayko et al., Nature Med. **3** (10), 1137-1140 (1997)).

Während diese Gentransfermethode vollkommen ungeeignet ist, um z.B. solide Tumoren oder Metastasen *in vivo* zu erreichen und zu therapieren, könnten hier LMW-PEI-Ribozym-Komplexe eine hervorragende Alternative bieten. Aus Vordaten anderer Gruppen ist bekannt, dass LMW-PEI sehr effizient mit geringer Toxizität die zelluläre Aufnahme von Desoxyribonukleinsäuren vermittelt.

Für die hier beschriebenen Experimente wurden die gegen FGF-BP gerichteten chemisch synthetisierten Ribozyme (1,0 und 0,1 ug; ca. 10 bzw. 1 nmol Endkonzentration) verwendet und mittels LMW-PEI in ME-180 Tumorzellen eingebracht. Die zwei unterschiedlichen Ribozymmengen wurden darüber hinaus in zwei verschiedenen Verhältnissen mit LMW-PEI komplexiert (P/N-Verhältnisse von 1:13 und 1:53). Diese Ribozym-PEI-Komplexe wurden für 4 Stunden in 10 ml serumfreiem IMDM-Medium (Firma PAA) auf ca. $1x10^6$ ME-180 Zellen, die als Monolayer auf 10 cm Zellkulturschalen wuchsen, gegeben. Als Kontrolle für unspezifische Effekte wurden ME-180 Zellen mit der gleichen Menge LMW-PEI ohne komplexiertes Ribozym behandelt. Danach wurde das Transfektionsmedium aspiriert und die Zellen wurden wieder auf normales Wachstumsmedium (IMDM plus 10 % FCS) gesetzt. Zu verschiedenen Zeitpunkten nach Beginn der Transfektion (6 - 36 Stunden) wurden die Zellen lysiert und die gesamte zelluläre RNS nach Standardprotokoll (Trizol-Reagenz, Firma Sigma) isoliert. Die so gewonnene RNS wurde mittels Northern-blot analysiert, wobei als Sonde eine mittels $^{32}P$ radioaktiv markierte FGF-BP cDNA eingesetzt wurde. Die Detektion von FGF-BP mRNS wurde mit Hilfe von Autoradiogrammen und Phosphorimager dargestellt und quantifiziert. Wie aus den Fig. 3 - 5 hervorgeht, führte die Transfektion mit LMW-PEI-Ribozymen zu einer sehr starken Ribozym-spezifischen Abnahme der FGF-BP Genexpression.

Die maximale Reduktion betrug ca. 70 % verglichen mit den Kontrollzellen. Beide Ribozym-PEI-Verhältnisse von 1:13 und 1:53 zeigten ähnliche Effektivität. Interessanterweise war die niedrigere Ribozym-Dosis von 0,1 µg in beiden PEI-Verhältnissen etwas wirksamer, so dass man davon ausgehen kann, dass auch noch niedrigere Dosen bereits sehr effizient sein können.

Diese ausgeprägte Inhibition der FGF-BP Genexpression um maximal 70 % nach 24 Stunden ist noch bemerkenswerter, wenn man sie im Kontext von Vergleichsuntersuchungen sieht. Wir haben in transienten Transfektionsstudien mit verschiedenen Gentransfervektoren (z.B. Plasmid-DNS, adenovirale Vektoren, verschiedene Lipidvesikel) weder mit Ribozymen noch mit DNS-Oligonukleotiden Hemmungen von mehr als 25 % beobachten können. Insbesondere haben wir mit chemisch stabilisierten RNS/DNS-Ribozym-Hybridmolekülen (werden als Goldstandard gesehen), die uns von der führenden Firma auf diesem Gebiet (Ribozyme Pharmaceuticals, Boulder, Colorado, USA) zur Verfügung gestellt wurden, keine Abnahme der Ziel-RNS nachweisen können (unpublizierte Daten).

Es lässt sich also festhalten, dass diese Kombination aus LMW-PEI und nicht-modifizierten RNS-Ribozymen ein neues und außerordentlich effektives Agens zur selektiven Reduktion der Expression von Genen nach Wahl ist.

**Beispiel 4:**

**Chemisch-syhthetisierte, nicht-modifizierte, mit LMW-PEI komplexierte RNS-Ribozyme führen nach intraperitonealer Applikation in athymischen Nacktmäusen zur spezifischen Hemmung des Wachstums von menschlichen Melanomxenotransplantaten**

Nachdem in den ersten drei Beispielen die Stabilisierung von verschiedenen RNS-Molekülen durch LMW-PEI sowie die biologische Aktivität von mit LMW-PEI komplexierten Ribozymen in menschlichen Tumorzellen in Zellkulturexperimenten gezeigt wurde, wurde untersucht, ob die systemische Applikation von mit LMW-PEI komplexierten Ribozymen in athymischen Nacktmäusen das Wachstum menschlicher Tumorzellxenotransplantaten hemmen kann.

Zu-diesem Zweck wurden chemisch synthetisierte Hammerhead-Ribozyme, die gegen die humane mRNS eines Heparin-bindenden-Wachstumsfaktors (Pleiotrophin = PTN) gerichtet sind, verwendet (Czubayko et al., PNAS **93**, 14753-14758 (1996)). Das PTN Protein wird in vielen menschlichen Tumoren und davon abgeleiteten Tumorzellinien exprimiert. Die Funktionalität der Ribozyme gegen PTN konnte in früheren Experimenten dadurch nachgewiesen werden, dass nach stabiler Transfektion der eukaryontischen Ribozymexpressionsvektoren in Tumorzellen (1205 Melanomzellinie) das Zielgen auf mRNS- und auf Proteinebene deutlich reduziert wurde (Czubayko et al., PNAS 93, 14753-14758 (1996)). Weiterhin war das Wachstum von 1205 Tumorzellxenotransplantaten in athymischen Nacktmäusen in den Ribozym-transfizierten Zellinien deutlich reduziert. Eine Reduktion der PTN-Proteinmenge um 25 % führte bereits zu einer Hemmung des Tumorwachstums in Nacktmäusen um ca. 33 %. Diese Vordaten machen dieses Tumormodell besonders geeignet zum Nachweis der Effizienz neuer Gentargetingmethoden *in vivo,* da aus der Hemmung des Tumorwachstums direkt auf das Ausmaß der Ribozymvermittelten Reduktion der PTN-Expression geschlossen werden kann.

Während stabile und transiente Plasmid-DNS Gentransfermethoden vollkommen ungeeignet sind, um z.B. solide Tumoren oder Metastasen *in vivo* zu erreichen und zu therapieren, könnten hier LMW-PEI-Ribozym-Komplexe eine hervorragende Alternative bieten.

In Vorversuchen in der Zellkultur wurde zuerst festgestellt, dass ein Ribozym / LMW-PEI Verhältnis von 1: 66,66 in 1205 Zellen optimale Transfektionergebnisse ergibt. Für den Tierversuch wurde eine Dosis von 8 µg Ribozym pro Injektion pro Maus festgelegt. Dies ergibt bei einem Gesamtgewicht von 20 g pro Maus eine Ribozym-Dosis von 0,4 mg/kg Körpergewicht. Diese Dosis sollte alle zwei Tage über drei Wochen den Versuchstieren intraperitoneal (i. p.) appliziert werden. Die Ribozym / LMW-PEI Komplexe wurden entsprechend Beispiel 1 jeweils am Morgen vor der Injektion frisch hergestellt.

Für den Tierversuch wurden in insgesamt 15 Versuchstieren jeweils zwei 1205 Tumorzellxenotransplantate in den Flanken der Tiere durch subkutane Injektion von $1x10^6$ Tumorzellen induziert. Nachdem die Tumore nach drei Tagen zu einer sichtbaren und tastbaren Grösse von ca. 2x2 mm herangewachsen waren, wurden die Tiere zufällig in eine Behandlungsgruppe und eine Kontrollgruppe eingeteilt. In der Behandlungsgruppe wurden 8 Tieren Ribozym/ LMW-PEI Komplexe (N/P Verhältnis = 1:66,66; 8 µg Ribozym / Injektion) im Abstand von 2 Tagen i.p. injiziert. In der Kontrollgruppe wurden 7 Tieren die gleiche Menge LMW-PEI (72 µg pro Injektion) ohne Ribozym im gleichen Abstand i.p. injiziert. Nach insgesamt 7 Injektionen wurden drei Tage nach der letzten Injektion die Tiere getötet, die subkutanen Tumoren entnommen und das Tumorvolumen bestimmt. In der Kontrollgruppe (7 Tiere = 14 Tumore) ergab sich eine mittlere Tumorgrösse von $2061 \pm 481$ $mm^3$ (Mittelwert und Standardabweichung), während in der Behandlungsgruppe (8 Tiere = 16 Tumore) eine mittlere Tumorgrösse von $1066 \pm 195$ $mm^3$ (Mittelwert und Standardabweichung) vorlag. Diese spezifische Ribozymvermittelte Hemmung des 1205 Tumorxenotransplantat-Wachstums um 48 % war im ungepaarten t-Test signifikant ($p< 0,05$).

Diese ausgeprägte Hemmung des 1205-Tumorwachstums um ca. 50 % nach 20 Tagen ist noch bemerkenswerter, wenn man sie im Kontext von Vergleichsuntersuchungen sieht. Wir haben in ähnlichen Tierversuchen mit chemisch stabilisierten RNS/DNS-Ribozym-Hybridmolekülen (werden als Goldstandard gesehen), die uns von der führenden Firma auf diesem Gebiet (Ribozyme Pharmaceuticals, Boulder, Colorado, USA) zur Verfügung gestellt wurden, lediglich eine Abnahme des 1205-Tumorwachstums um ca. 20 % nachweisen können (unpublizierte Daten).

Es lässt sich also festhalten, dass diese Kombination aus LMW-PEI und nichtmodifizierten RNS-Ribozymen ein neues und außerordentlich effektives Agens zur selektiven Reduktion der Expression von Genen in vivo in Tierversuchen ist.

**Erläuterung der Abbildungen:**

**Fig. 1** FGF-BP RNA (800 Nucleotide) - Degradation in 20 ng/ml RNaseA (plus / minus LMW-Polyethylenimin (PEI) - Komplexierung): Während bei der Inkubation mit RNase A (Endkonzentration 20 ng/ml) die nichtkomplexierte

RNS erwartungsgemäß bereits nach 15 Minuten fast komplett abgebaut ist, sind von der PEI-komplexierten RNS auch nach 2 Stunden Inkubation noch mehr als 60 % der Moleküle intakt.

**Fig. 2** FGF-BP RNA (800 Nucleotide) - Degradation in 1 % FCS ( plus / minus LMW-Polyethylenimin (PEI) - Komplexierung):
Eine sehr ähnliche Kinetik wie in Fig. 1 ergibt sich auch nach der Inkubation mit fötalem Kälberserum (Fig. 2). Hier ist die nicht-komplexierte RNS nach ca. 30 Minuten nach der Inkubation mit 1 % fötalem Kälberserum komplett abgebaut, während die PEI-komplexierte RNS auch nach 120 Minuten noch vollständig intakt ist. Auch bei Erhöhung der Konzentration auf 40 % fötales Kälberserum ist die PEI-komplexierte RNS gegenüber der nicht-komplexierten RNS deutlich stabiler. Die Ergebnisse waren identisch für die verschieden langen RNS-Moleküle. Die Daten der Graphiken wurden aus zwei unabhängigen Experimenten gemittelt.

**Fig. 3** FGF-BP Ribozyme komplexiert mit niedermolekularem PEI inhibieren FGF-BP mRNA Expression in ME-180 Plattenepithelkarzinomzellen nach transienter Transfektion.

**Fig. 4** FGF-BP Ribozyme komplexiert mit niedermolekularem PEI (N/P-Verhältnis 1:13) inhibieren FGF-BP mRNA Expression in ME-180 Plattenepithelkarzinomzellen nach transienter Transfektion.

**Fig. 5** FGF-BP Ribozyme komplexiert mit niedermolekularem PEI (N/P-Verhältnis 1:53) inhibieren FGF-BP mRNA Expression in ME- 180 Plattenepithelkarzinomzellen nach transienter Transfektion.

Wie aus den Fig. 3 - 5 hervorgeht, führte die Transfektion mit LMW-PEI-Ribozymen zu einer sehr starken Ribozym-spezifischen Abnahme der FGF-BP Genexpression.

Die maximale Reduktion betrug ca. 70 % verglichen mit den Kontrollzellen. Beide Ribozym-PEI-Verhältnisse von 1:13 und 1:53 zeigten ähnliche Effektivität. Interessanterweise war die niedrigere Ribozym-Dosis von 0,1 µg in beiden PEI-Verhältnissen etwas wirksamer, so daß man davon ausgehen kann, dass auch noch niedrigere Dosen bereits sehr effizient sein können.

**Fig. 6** Pleiotrophin (PTN) Ribozyme komplexiert mit niedermolekularem PEI (N/P-Verhältnis 1:66,66) inhibieren das Wachstum von humanen Melanom-Tumorzelltransplantaten (1205 Zellen) in athymischen Nacktmäusen nach systemischer Applikation.

1205 Tumorzellen (1x$10^6$ Zellen/Injektion) wurden in die Flanken von athymischen Nacktmäusen subkutan injiziert, und entweder mit PTN-Ribozymen komplexiert mit LMW-PEI (8µg Ribozym / Injektion) intraperitoneal (i.p.) im Abstand von zwei Tagen injiziert (Behandlungsgruppe = 8 Tiere), oder nur mit LMW-PEI im gleichen Abstand behandelt (Kontrollgruppe = 7 Tiere). Nach 20 Tagen zeigte sich eine spezifische Ribozym-vermittelte Hemmung des Tumorwachstums um ca. 50 %.

## Patentansprüche

1. Verwendung eines Komplexes von Ribozym mit Polyethylenimin (PEI), welches gegebenenfalls mit daran gekoppelten hydrophilen Polymeren modifiziert ist, zum Schutz des Ribozyms gegen enzymatischen oder nichtenzymatischen, intra- oder extrazellulären Abbau, wobei das PEI ein Molekulargewicht im Bereich von 700 bis 25.000 Dalton aufweist.

2. Verwendung gemäß Anspruch 1,
   **dadurch gekennzeichnet,**
   **dass** das Ribozym vor dem intra- oder extrazellulären Abbau durch Nukleasen geschützt wird.

3. Verwendung gemäß einem der Ansprüche 1 oder 2,
   **dadurch gekennzeichnet,**
   **dass** das Ribozym in aktiver Form nach dessen Einschleusung in die Zelle intrazellulär aus dem Komplex freigesetzt wird und biologische Funktionen ausüben kann.

4. Verwendung gemäß einem der Ansprüche 1 bis 3,
   **dadurch gekennzeichnet,**
   **dass** das Ribozym aus der Gruppe ausgewählt wird, die aus Hammerhead-Ribozym, Hairpin-Ribozym, HDV-Ribozym, RNaseP, Intron-Gruppe 1-Ribozym, Intron-Gruppe 2-Ribozym und Mischungen davon besteht.

5. Verwendung gemäß einem der Ansprüche 1 bis 4,
**dadurch gekennzeichnet,**
**dass** das Ribozym 20 bis 600 Nukleotide, vorzugsweise 35 bis 80 Nukleotide aufweist.

6. Verwendung gemäß einem der Ansprüche 1 bis 5,
**dadurch gekennzeichnet,**
**dass** das Ribozym ein unmodifiziertes Ribozym ist.

7. Verwendung gemäß einem der Ansprüche 1 bis 6,
**dadurch gekennzeichnet,**
**dass** das PEI nicht mit daran gekoppelten hydrophilen Polymeren modifiziert ist .

8. Verwendung gemäß einem der Ansprüche 1 bis 7,
**dadurch gekennzeichnet,**
**dass** das PEI mit einem oder mehreren daran gekoppelten hydrophilen Polymeren modifiziert ist.

9. Verwendung gemäß einem der Ansprüche 1 bis 8,
**dadurch gekennzeichnet,**
**dass** das hydrophile Polymer mit einem oder mehreren daran gekoppelten PEI modifiziert ist.

10. Verwendung gemäß einem der Ansprüche 1 bis 6 und 8 bis 9,
**dadurch gekennzeichnet,**
**dass** das hydrophile Polymer Polyethylenglykol (PEG) ist.

11. Verwendung gemäß einem der Ansprüche 1 bis 10,
**dadurch gekennzeichnet,**
**dass** das PEI ein Molekulargewicht von 1.400 bis 25.000 Da, insbesondere 1.600 bis 15.000 Da, aufweist.

12. Verwendung gemäß einem der Ansprüche 1 bis 11,
**dadurch gekennzeichnet,**
**daß** das molare Verhältnis der Stickstoffatome im PEI zu den Phosphoratomen in dem Ribozym (N/P-Wert) 1 bis 100, vorzugsweise 2 bis 40, insbesondere 3 bis 10 beträgt.

13. Verwendung gemäß einem der Ansprüche 1 bis 12,
**dadurch gekennzeichnet,**
**dass** das PEI durch einen zellulären Liganden modifiziert ist.

14. Verwendung gemäß einem der Ansprüche 1 bis 13,
**dadurch gekennzeichnet,**
**dass** die Ribozym-Konzentration 0,01 - 1000 µg/ml, vorzugsweise 1 - 100 µg/ml, besonders bevorzugt 10 - 40 µg/ml, beträgt.

15. Komplex aus Ribozym und Polyethylenimin (PEI),
**dadurch gekennzeichnet,**
**dass** das PEI gegebenenfalls mit daran gekoppelten hydrophilen Polymeren modifiziert ist; und daß das PEI ein Molekulargewicht im Bereich von 700 bis 25.000 Dalton aufweist.

16. Komplex gemäß Anspruch 15,
**dadurch gekennzeichnet,**
**dass** das Ribozym aus der Gruppe ausgewählt wird, die aus Hammerhead-Ribozym, Hairpin-Ribozym, HDV-Ribozym, RNaseP, Intron-Gruppe 1-Ribozym, Intron-Gruppe 2-Ribozym und Mischungen davon besteht.

17. Komplex gemäß Anspruch 15 oder 16,
**dadurch gekennzeichnet,**
**dass** das Ribozym 20 bis 600 Nukleotide, vorzugsweise 35 bis 80 Nukleotide, aufweist.

18. Komplex gemäß einem der Ansprüche 15 bis 17,
**dadurch gekennzeichnet,**

**dass** das Ribozym ein unmodifiziertes Ribozym ist.

**19.** Komplex gemäß einem der Ansprüche 15 bis 18,
**dadurch gekennzeichnet,**
**daß** das PEI nicht mit daran gekoppelten hydrophilen Polymeren modifiziert ist.

**20.** Komplex gemäß einem der Ansprüche 15 bis 18,
**dadurch gekennzeichnet,**
**dass** das PEI mit einem oder mehreren daran gekoppelten hydrophilen Polymeren modifiziert ist.

**21.** Komplex gemäß einem der Ansprüche 15 bis 18 und 20,
**dadurch gekennzeichnet,**
**dass** das hydrophile Polymer mit einem oder mehreren daran gekoppelten PEI modifiziert ist.

**22.** Komplex gemäß einem der Ansprüche 15 bis 18 und 20 bis 21,
**dadurch gekennzeichnet,**
**dass** das hydrophile Polymer Polyethylenglykol (PEG) ist.

**23.** Komplex gemäß einem der Ansprüche 15 bis 22,
**dadurch gekennzeichnet,**
**dass** das PEI ein Molekulargewicht von 1.400 bis 25.000 Da, insbesondere 1.600 bis 15.000 Da, aufweist.

**24.** Komplex gemäß einem der Ansprüche 15 bis 23,
**dadurch gekennzeichnet,**
**dass** das molare Verhältnis der Stickstoffatome im PEI zu den Phosphoratomen im Ribozym (N/P-Wert) 1 bis 100, vorzugsweise 2 bis 40, insbesondere 3 bis 10 beträgt.

**25.** Komplex gemäß einem der Ansprüche 15 bis 23,
**dadurch gekennzeichnet,**
**dass** das PEI durch einen zellulären Liganden modifiziert ist.

**26.** Verfahren zur Herstellung eines Komplexes gemäß einem der Ansprüche 15 bis 25,
**dadurch gekennzeichnet,**
**dass** das Ribozym mit dem gegebenenfalls durch einen zellulären Liganden oder gegebenenfalls durch ein weiteres Polymer modifizierten PEI durch Mischen von verdünnten Lösungen komplexiert wird.

**27.** Verfahren gemäß Ansprüch 26,
**dadurch gekennzeichnet,**
**dass** die Ribozym-Konzentration 0,01 - 1000 µg/ml, vorzugsweise 1 - 100 µg/ml, besonders bevorzugt 10 - 40 µg/ml, beträgt.

**28.** Pharmazeutische Zusammensetzung enthaltend eine wirksame Menge mindestens eines Komplexes gemäß einem der Ansprüche 15 bis 25 und einen oder mehrere physiologisch unbedenkliche Träger.

**29.** Zusammensetzung gemäß Anspruch 28,
**dadurch gekennzeichnet,**
**dass** das Ribozym in einer für Zellen oder für den jeweiligen Organismus subtoxischen oder zumindest bei mehrstündiger Behandlung noch nicht dauerhaft zellschädigenden Konzentration vorliegt.

**30.** Zusammensetzung gemäß Anspruch 28 oder 29 zur Verwendung bei der Gentherapie.

**31.** Verwendung eines Komplexes gemäß einem der Ansprüche 15 bis 25 zum Schutz von Ribozymen gegen enzymatischen oder nicht-enzymatischen Abbau bei der Lagerung oder beim Transport der Ribozyme, modifiziert oder nicht modifiziert, in wässrigen Lösungen, oder bei der Verwendung von Ribozymen, modifiziert oder nicht modifiziert, in Laborexperimenten.

**32.** Verwendung eines Komplexes gemäß einem der Ansprüche 15 bis 25 zur Herstellung einer pharmazeutischen Zusammensetzung zur Einschleusung von Ribozymen in Zellen, vorzugsweise in eukaryontische Zellen und zu

deren intrazellulärer Freisetzung in biologisch aktiver Form.

**Claims**

1. Use of a complex of ribozyme with polyethyleneimine (PEI) which is optionally modified with a hydrophilic polymer coupled thereto, for protecting the ribozyme from enzymatic or non-enzymatic, intra- or extracellular degradation, where the PEI has a molecular weight in the range from 700 to 25 000 dalton.

2. Use according to Claim 1, **characterized in that** the ribozyme is protected from intra- or extracellular degradation by nucleases.

3. Use according to either of Claims 1 or 2, **characterized in that** the ribozyme is released in active form after its introduction into the cell intracellularly from the complex and is able to perform biological functions.

4. Use according to any of Claims 1 to 3, **characterized in that** the ribozyme is selected from the group consisting of hammerhead ribozyme, hairpin ribozyme, HDV ribozyme, RNaseP, intron group 1 ribozyme, intron group 2 ribozyme and mixtures thereof.

5. Use according to any of Claims 1 to 4, **characterized in that** the ribozyme has from 20 to 600 nucleotides, preferably 35 to 80 nucleotides.

6. Use according to any of Claims 1 to 5, **characterized in that** the ribozyme is an unmodified ribozyme.

7. Use according to any of Claims 1 to 6, **characterized in that** the PEI is not modified with hydrophilic polymers coupled thereto.

8. Use according to any of Claims 1 to 7, **characterized in that** the PEI is modified with one or more hydrophilic polymers coupled thereto.

9. Use according to any of Claims 1 to 8, **characterized in that** the hydrophilic polymer is modified with one or more PEI coupled thereto.

10. Use according to any of Claims 1 to 6 and 8 to 9, **characterized in that** the hydrophilic polymer is polyethylene glycol (PEG).

11. Use according to any of Claims 1 to 10, **characterized in that** the PEI has a molecular weight of from 1400 to 25 000 Da, in particular 1600 to 15 000 Da.

12. Use according to any of Claims 1 to 11, **characterized in that** the molar ratio of the nitrogen atoms in the PEI to the phosphorus atoms in the ribozyme (N/P value) is from 1 to 100, preferably 2 to 40, in particular 3 to 10.

13. Use according to any of Claims 1 to 12, **characterized in that** the PEI is modified by a cellular ligand.

14. Use according to any of Claims 1 to 13, **characterized in that** the ribozyme concentration is 0.01-1000 µg/ml, preferably 1-100 pg/ml, particularly preferably 10-40 µg/ml.

15. Complex of ribozyme and polyethyleneimine (PEI), **characterized in that** the PEI is optionally modified with hydrophilic polymers coupled thereto, and **in that** the PEI has a molecular weight in the range from 700 to 25 000 dalton.

16. Complex according to Claim 15, **characterized in that** the ribozyme is selected from the group consisting of hammerhead ribozyme, hairpin ribozyme, HDV ribozyme, RNaseP, intron group 1 ribozyme, intron group 2 ribozyme and mixtures thereof.

17. Complex according to Claim 15 or 16, **characterized in that** the ribozyme has from 20 to 600 nucleotides, preferably 35 to 80 nucleotides.

**18.** Complex according to any of Claims 15 to 17, **characterized in that** the ribozyme is an unmodified ribozyme.

**19.** Complex according to any of Claims 15 to 18, **characterized in that** the PEI is not modified with hydrophilic polymers coupled thereto.

**20.** Complex according to any of Claims 15 to 18, **characterized in that** the PEI is modified with one or more hydrophilic polymers coupled thereto.

**21.** Complex according to any of Claims 15 to 18 and 20, **characterized in that** the hydrophilic polymer is modified with one or more PEI coupled thereto.

**22.** Complex according to any of Claims 15 to 18 and 20 to 21, **characterized in that** the hydrophilic polymer is polyethylene glycol (PEG).

**23.** Complex according to any of Claims 15 to 22, **characterized in that** the PEI has a molecular weight of from 1400 to 25 000 Da, in particular 1600 to 15 000 Da.

**24.** Complex according to any of Claims 15 to 23, **characterized in that** the molar ratio of the nitrogen atoms in the PEI to the phosphorus atoms in the ribozyme (N/P value) is from 1 to 100, preferably 2 to 40, in particular 3 to 10.

**25.** Complex according to any of Claims 15 to 23, **characterized in that** the PEI is modified by a cellular ligand.

**26.** Method for preparing a complex according to any of Claims 15 to 25, **characterized in that** the ribozyme is complexed with the PEI, which is modified optionally by a cellular ligand or optionally by a further polymer, by mixing dilute solutions.

**27.** Method according to Claim 26, **characterized in that** the ribozyme concentration is 0.01-1000 µg/ml, preferably 1-100 µg/ml, particularly preferably 10-40 µg/ml.

**28.** Pharmaceutical composition comprising an effective amount of at least one complex according to any of Claims 15 to 25 and one or more physiologically acceptable carrier.

**29.** Composition according to Claim 28, **characterized in that** the ribozyme is present in a concentration which is subtoxic for cells or for the particular organism or which is not permanently damaging for cells at least on treatment for a plurality of hours.

**30.** Composition according to Claim 28 or 29 for use in gene therapy.

**31.** Use of a complex according to any of Claims 15 to 25 for protecting ribozymes from enzymatic or non-enzymatic degradation on storage or on transport of the ribozymes, modified or unmodified, in aqueous solutions, or on use of ribozymes, modified or unmodified, in laboratory experiments.

**32.** Use of a complex according to any of Claims 15 to 25 for producing a pharmaceutical composition for the introduction of ribozymes into cells, preferably into eukaryotic cells, and for the intracellular release thereof in biologically active form.

## Revendications

**1.** Utilisation d'un complexe de ribozyme avec du polyéthylènimine (PEI), qui est éventuellement modifié avec des polymères qui y sont couplés, pour la protection du ribozyme contre une dégradation enzymatique ou non enzymatique, intra ou extracellulaire, le PEI présentant un poids moléculaire dans la gamme entre 700 et 25 000 Daltons.

**2.** Utilisation selon la revendication 1, **caractérisée en ce que** le ribozyme est protégé par des nucléases avant la dégradation intra ou extracellulaire.

**3.** Utilisation selon la revendication 1 ou 2, **caractérisée en ce que** le ribozyme est libéré du complexe au plan

intracellulaire sous sa forme active après son introduction dans la cellule et peut exercer des fonctions biologiques.

4. Utilisation selon l'une des revendications 1 à 3, **caractérisée en ce que** le ribozyme est choisi dans le groupe constitué du ribozyme en tête de marteau, du ribozyme en épingle à cheveux, du ribozyme du VHD, de la RnaseP, du ribozyme de l'intron du groupe &, du ribozyme de l'intron du groupe 2 et de mélanges de ceux-ci.

5. Utilisation selon l'une des revendications 1 à 4, **caractérisée en ce que** le ribozyme présente de 20 à 600 nucléotides, de préférence de 35 à 80 nucléotides.

6. Utilisation selon l'une des revendications 1 à 5, **caractérisée en ce que** le ribozyme est un ribozyme non modifié.

7. Utilisation selon l'une des revendications 1 à 6, **caractérisée en ce que** le PEI n'est pas modifié avec des polymères hydrophiles qui y sont couplés.

8. Utilisation selon l'une des revendications 1 à 7, **caractérisée en ce que** le PEI est modifié par un ou plusieurs polymères hydrophiles qui y sont couplés.

9. Utilisation selon l'une des revendications 1 à 8, **caractérisée en ce que** le polymère hydrophile est modifié par un ou plusieurs PEI qui y sont couplés.

10. Utilisation selon l'une des revendications 1 à 6 et 8 à 9, **caractérisée en ce que** le polymère hydrophile est un polyéthylène glycol (PEG).

11. Utilisation selon l'une des revendications 1 à 10, **caractérisée en ce que** le PEI présente un poids moléculaire de 1400 à 25 000 Da, en particulier de 1600 à 15 000 Da.

12. Utilisation selon l'une des revendications 1 à 11, **caractérisée en ce que** le rapport molaire entre les atomes d'azote dans le PEI et les atomes de phosphore dans le ribozyme (valeur N/P) est de 1 à 100, de préférence de 2 à 40, en particulier de 3 à 10.

13. Utilisation selon l'une des revendications 1 à 12, **caractérisée en ce que** le PEI est modifié par un ligand cellulaire.

14. Utilisation selon l'une des revendications 1 à 13, **caractérisée en ce que** la concentration de ribozyme s'élève à 0,01-1000 µg/ml, de préférence à 1-100 µg/ml, de façon particulièrement préférée à 10-40 µg/ml.

15. Complexe constitué de ribozyme et de polyéthylèneimine (PEI), **caractérisé en ce que** le PEI est éventuellement modifié par des polymères hydrophiles qui y sont couplés, et **en ce que** le PEI présente un poids moléculaire dans la gamme de 700 à 25 000 Daltons.

16. Complexe selon la revendication 15, **caractérisé en ce que** le ribozyme est choisi dans le groupe constitué du ribozyme en tête de marteau, du ribozyme en épingle à cheveux, du ribozyme du VHD, de la RNaseP, du ribozyme de l'intron du groupe 1, du ribozyme de l'intron du groupe 2 et de mélanges de ceux-ci.

17. Complexe selon la revendication 15 ou 16, **caractérisé en ce que** le ribozyme présente de 20 à 600 nucléotides, de préférence de 35 à 80 nucléotides.

18. Complexe selon la revendication 15 à 17, **caractérisé en ce que** le ribozyme est un ribozyme non modifié.

19. Complexe selon la revendication 15 à 18, **caractérisé en ce que** le PEI n'est pas modifié par des polymères hydrophiles qui y sont couplés.

20. Complexe selon la revendication 15 à 18, **caractérisé en ce que** le PEI est modifié par un ou plusieurs polymères hydrophiles qui y sont couplés.

21. Complexe selon la revendication 15 à 18 et 20, **caractérisé en ce que** le polymère hydrophile est modifié avec un ou plusieurs PEI qui y sont couplés.

22. Complexe selon la revendication 15 à 18 et 20 à 21, **caractérisé en ce que** le polymère hydrophile est un polyéthylène glycol (PEG).

**23.** Complexe selon la revendication 15 à 22, **caractérisé en ce que** le PEI présente un poids moléculaire de 1400 à 25 000 Da, en particulier de 1600 à 15 000 Da.

**24.** Complexe selon la revendication 15 à 23, **caractérisé en ce que** le rapport molaire entre les atomes d'azote dans le PEI et les atomes de phosphore dans le ribozyme (valeur N/P) est de 1 à 100, de préférence de 2 à 40, en particulier de 3 à 10.

**25.** Complexe selon l'une des revendications 15 à 23, **caractérisé en ce que** le PEI est modifié par un ligand cellulaire.

**26.** Procédé de fabrication d'un complexe selon l'une des revendications 15 à 25, **caractérisé en ce que** le ribozyme est complexé avec un PEI modifié éventuellement par un ligand cellulaire ou éventuellement par un autre polymère par le biais du mélange de solutions diluées.

**27.** Procédé selon la revendication 26, **caractérisé en ce que** la concentration de ribozyme s'élève à 0,01-1000 µg/ml, de préférence à 1-100 µg/ml, de façon particulièrement préférée à 10-40 µg/ml.

**28.** Composition pharmaceutique contenant une quantité efficace d'au moins un complexe selon l'une des revendications 15 à 25 et d'un ou plusieurs supports physiologiquement inoffensif.

**29.** Composition selon la revendication 28, **caractérisée en ce que** le ribozyme est présent dans une concentration non toxique pour les cellules ou l'organisme concerné ou au moins sans effet néfaste durable sur les cellules dans le cadre d'un traitement de plusieurs heures.

**30.** Composition selon la revendication 28 ou 29 pour une utilisation en thérapie génique.

**31.** Utilisation d'un complexe selon l'une des revendications 15 à 25 pour la protection de ribozymes contre une dégradation enzymatique ou non enzymatique lors du stockage ou du transport de ribozymes, modifiés ou non modifiés, dans des solutions aqueuses, ou lors de l'utilisation de ribozymes, modifiés ou non modifiés, dans des expériences en laboratoire.

**32.** Utilisation d'un complexe selon l'une des revendications 15 à 25 pour la fabrication d'une composition pharmaceutique en vue de l'introduction de ribozymes dans des cellules, de préférence des cellules eucaryotes, et de leur libération intracellulaire sous une forme biologiquement active.

Fig. 1

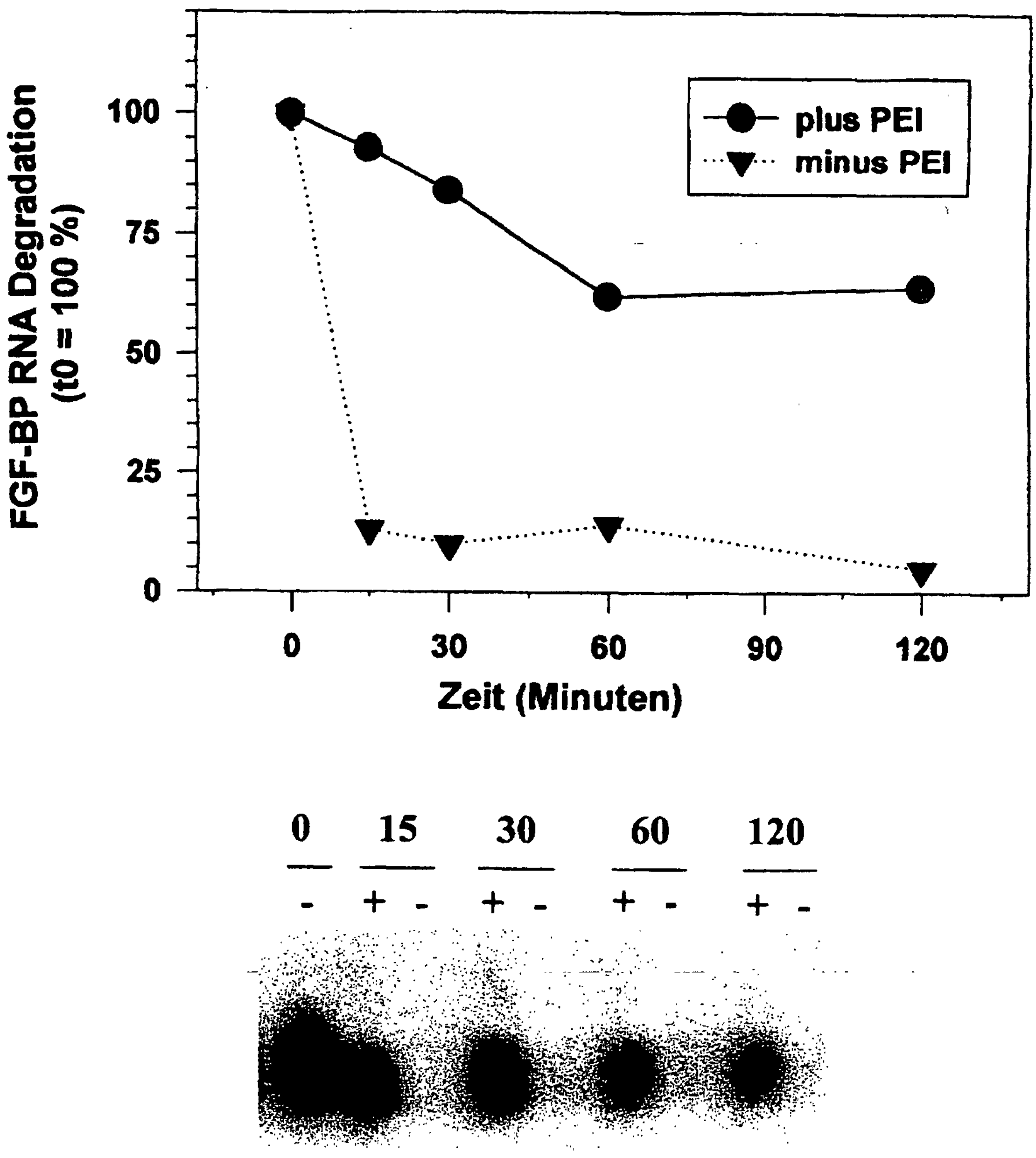
FGF-BP RNA Degradation
(t0 = 100 %)
100
75
50
25
0
plus PEI
minus PEI
0
30
60
90
120
Zeit (Minuten)
0
15
30
60
120
- + - + - + - + -

Fig. 2

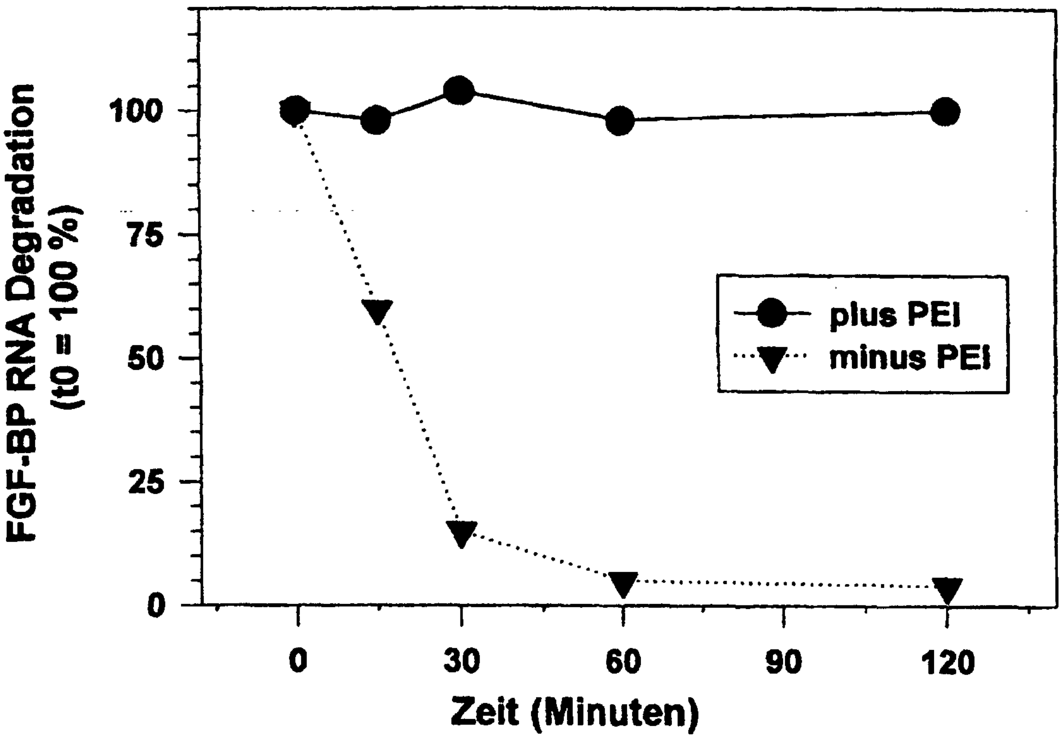
FGF-BP RNA Degradation
(t0 = 100 %)
100
75
50
25
0
plus PEI
minus PEI
0
30
60
90
120
Zeit (Minuten)

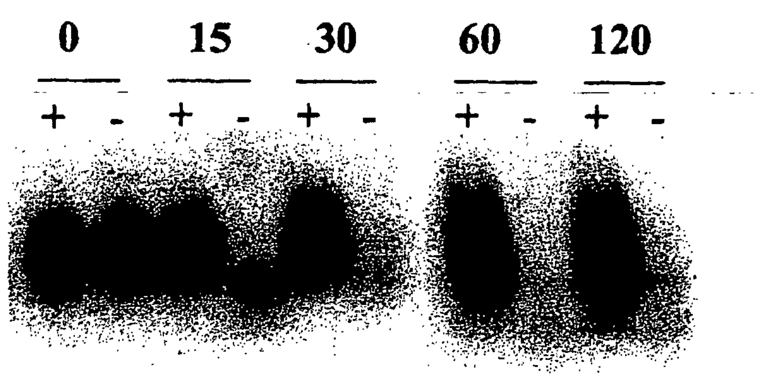
0
15
30
60
120
+
-
+
-
+
-
+
-
+
-

Fig. 3

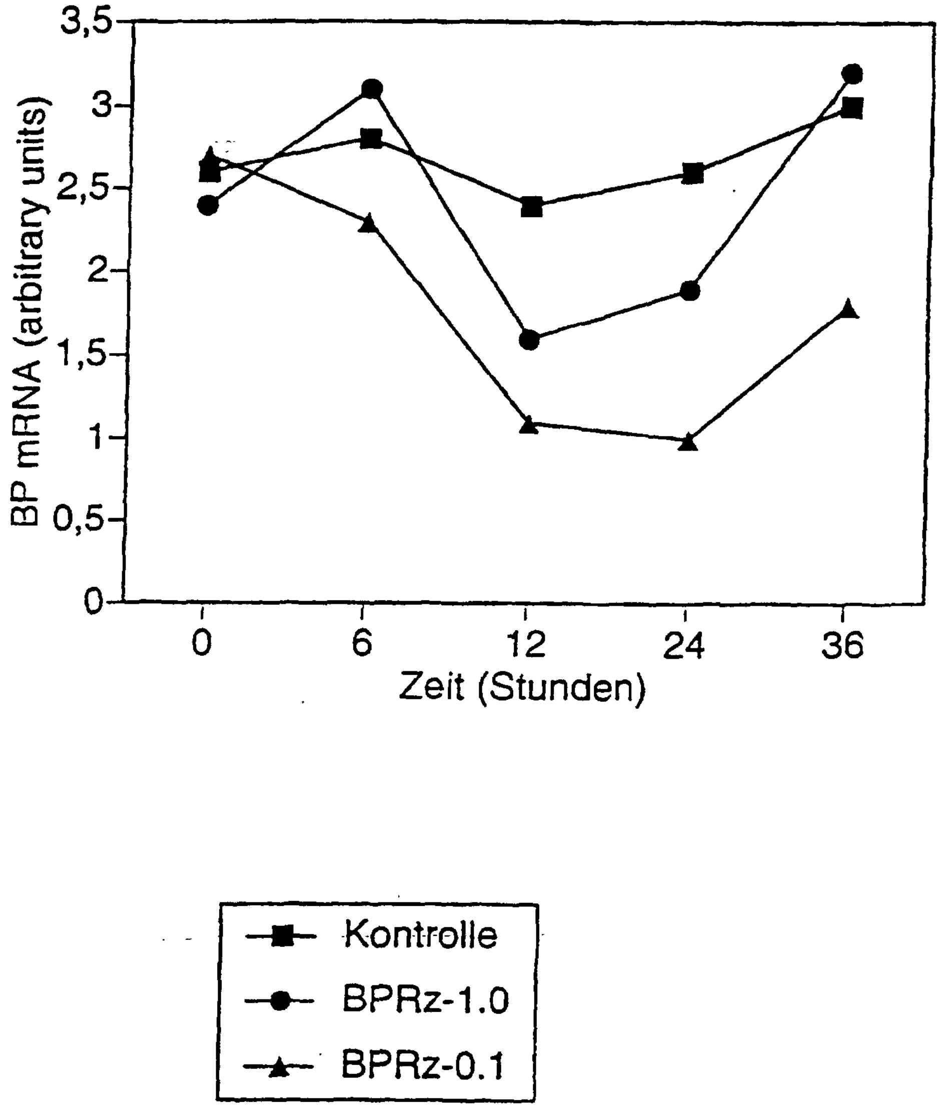
3,5
3
2,5
2
1,5
1
0,5
0
BP mRNA (arbitrary units)
0
6
12
24
36
Zeit (Stunden)
Kontrolle
BPRz-1.0
BPRz-0.1

Fig. 4

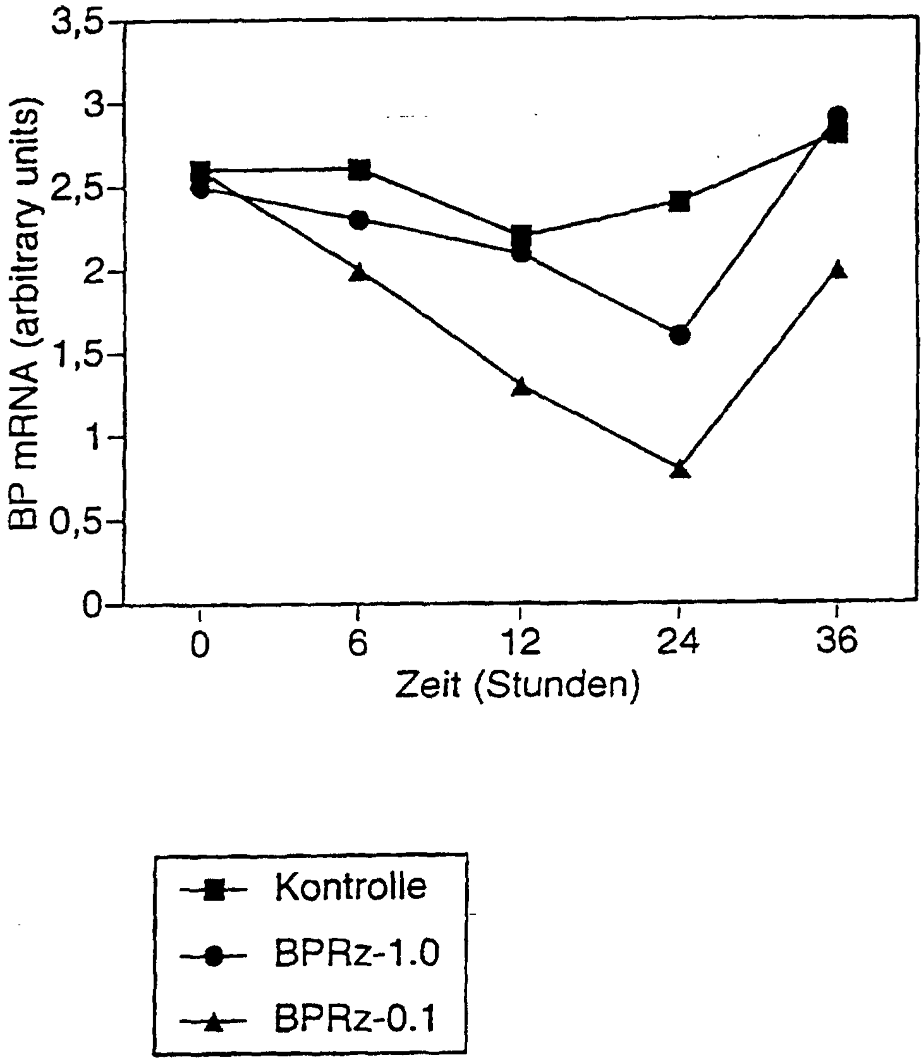
3,5
3
2,5
2
1,5
1
0,5
0
BP mRNA (arbitrary units)
0
6
12
24
36
Zeit (Stunden)
Kontrolle
BPRz-1.0
BPRz-0.1

Fig. 5

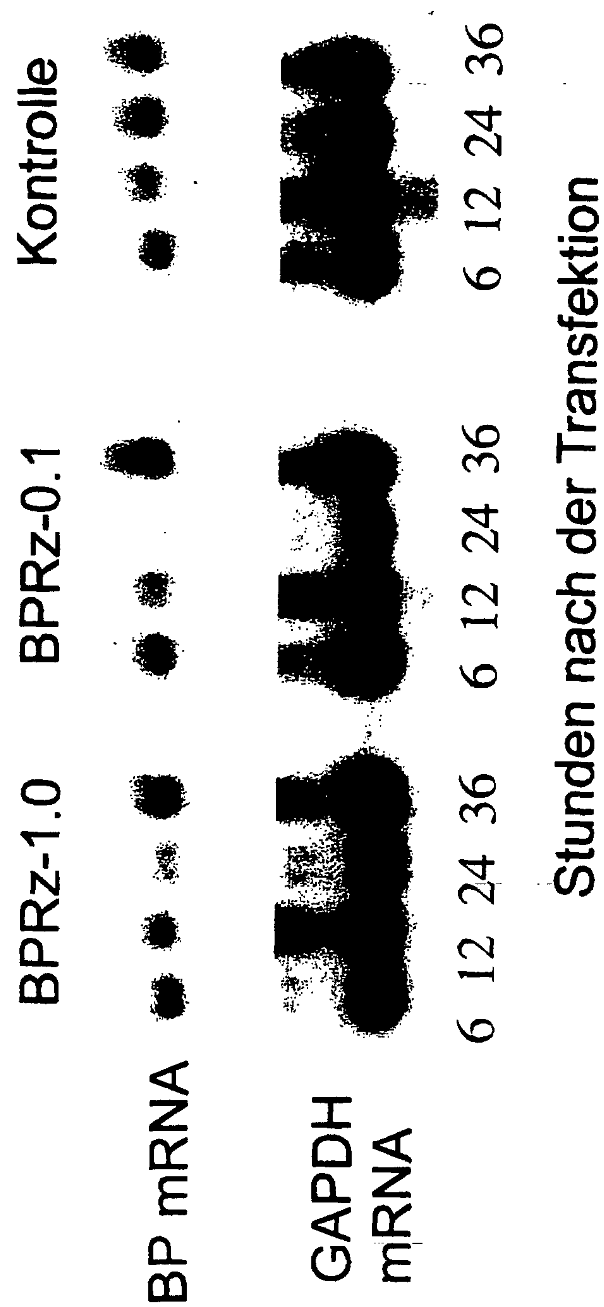
BPRz-1.0
BPRz-0.1
Kontrolle
BP mRNA
GAPDH mRNA
6 12 24 36
6 12 24 36
6 12 24 36
Stunden nach der Transfektion

Fig. 6

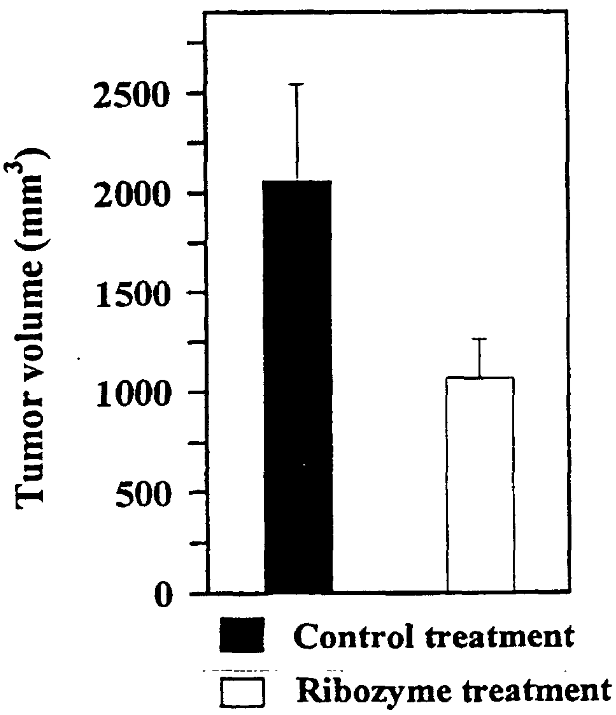

Tumor volume (mm$^3$)
2500
2000
1500
1000
500
0
Control treatment
Ribozyme treatment